# EUROPÄISCHE PATENTANMELDUNG

(11) **EP 1 332 748 A1**
(43) Veröffentlichungstag der Anmeldung: **06.08.2003**
(21) Anmeldenummer: 02000252.3
(22) Anmeldetag: 25.01.2002
(51) Int. Cl.: A61H 19/00

(54) **Gerät zur Stimulation des männlichen Gliedes**

(71) Anmelder: Haimerl, Michael, 94265 Patersdorf (DE)
(72) Erfinder: Haimerl, Michael, 94265 Patersdorf (DE)

(57) **Zusammenfassung**

Das in der Beschreibung und des Patentanspruches näher bezeichnete Partnergerät soll die Bewegung beim Geschlechtsverkehr unterstützen. Hier vor allem bei älteren Partnern und Behinderten.

Besonders hilfreich ist es aber bei erektiler Dysfunktion.

## Beschreibung

### Allgemeine Anwendung:

Vorliegendes Gerät ist ein Partnergerät. Das Gerät soll den evtl. Bewegungsmangel beim Geschlechtsverkehr unterstützen. Hier vor allem bei älteren oder behinderten Partnern. Außerdem kann es die erektile Dysfunktion bei sonst gesunden männlichen Partnern beheben.

### Beschreibung des Gerätes:

Das Gerät besteht aus einem geschmeidigen Rohr in Aluminium oder Kunststoff. Länge ca. 100cm Durchmesser ca.14mm. In diesem Rohr ist ein Kern aus Holz oder Metall, der die Schwingungen vom Aggregat überträgt. An beiden Enden des Kernes ist ein Gummi installiert. An diesen festen aber elastischen Gummi wird zum einen Ende das Pendelhubaggregat, mittels geeignetem Anschluß, installiert. Am anderen Ende ist ein elastischer Ring von ca. 50mm Durchmesser und 25mm Breite am Gummi befestigt. Diese beiden Gummienden machen die Schwingungen weich. Der Ring ist drehbar. Auf Grund der beiden Gummienden ist das Gerät auch sehr flexibel und in jede Richtung beweglich. Nur dieser Holzkern mit den Gummipuffern und dem angeschlossenen Ring wird bewegt. Die Hülle (Alurohr) bewegt sich nicht und dient dem Schutz vor Reibung an der Haut. Das Antriebsaggregat steht außerhalb der Reichweite des Paares und ist schallisoliert. Das Aggregat kann über einen konventionellen Kabelanschluß oder über einen Funkschalter gesteuert werden. Als Antrieb kann jeder handelsübliche Pendelhubmotor mit elektronischer Geschwindigkeitsregelung verwendet werden.

### Funktion:

In den elastischen Ring wird das männliche Glied geschoben. Der Ring wird sodann, in Schwingungen versetzt. Auf Grund der (variabel einstellbaren) Schwingungen wird das Blut in den Penismuskel gedrückt und er versteift sich in kurzer Zeit. Da der Penis zu etwa 75% frei ist kann er so problemlos in die Vagina eindringen. Das Gerät kann dann entweder einfach weggelegt werden oder es wird zur Bewegungsunterstützung und Luststeigerung weiter betrieben. Durch die Flexibilität sind die Partner in Ihrer Bewegungsfreiheit nicht eingeschränkt. Sie können so unbeschränkte Zeit und ohne Anstrengung zusammen sein. Ein weiterer positiver Effekt ist. Die Geschlechtsteile müssen nicht mehr (wie üblich) gestoßen werden, was gerade bei älteren Paaren oft wegen Trockenheit schmerzhaft ist.

Außerdem, sollten einige Paare Ihre erogenen Zonen wo anders haben, so kann das Gerät mit zusätzlichen Fühlern für den Anus bestückt werden.

Falls eine Zeichnung oder Foto erforderlich ist, sende ich es nach. Ich bitte um Ihre Information.

## Patentansprüche

1. Anspruch auf das Patent wird von mir als Erfinder erhoben, auf daß unten näher bezeichnete technische Gerät unter dem Sammelbegriff "Partnergerät" und dem speziellen Namen "Pallust" (Partnerlust)
Bestandteile des Gerätes:
Das Gerät besteht aus einem geschmeidigen Rohr in Aluminium oder Kunststoff. Länge ca. 100cm Durchmesser ca.14mm. (Das Rohr kann auch, je nach Erfordernis, kürzer oder langer sein. In diesem Rohr ist ein Kern aus Holz oder Metall, der die Schwingungen vom Aggregat überträgt. An beiden Enden des Kernes ist ein Gummi installiert. An diesen festen aber elastischen Gummi wird zum einen Ende das Pendelhubaggregat, mittels geeignetem Anschluß, installiert. Am anderen Ende ist ein elastischer Ring von ca. 50mm Durchmesser und 25mm Breite am Gummi befestigt. Der Ring ist drehbar. Das Aggregat kann über einen konventionellen Kabelanschluß oder über einen Funkschalter gesteuert werden. Als Antrieb kann jeder handelsübliche Pendelhubmotor mit elektronischer Geschwindigkeitsregelung verwendet werden.
